# EUROPÄISCHE PATENTSCHRIFT

(11) **EP 0 551 791 B1**
(45) Veröffentlichungstag und Bekanntmachung des Hinweises auf die Patenterteilung: **15.01.1997**
(21) Anmeldenummer: 92810940.4
(22) Anmeldetag: 02.12.1992
(51) Int. Cl.: A61F 2/38

(54) **Künstliches Kniegelenk**
Artificial knee joint
Articulation de genou artificielle

(30) Priorität: 14.01.1992 CH 88/92
(43) Veröffentlichungstag der Anmeldung: 21.07.1993
(73) Patentinhaber: Sulzer Orthopädie AG, 6340 Baar (CH)
(72) Erfinder: Moser, Walter, Dr., CH-3037 Herrenschwanden (CH); Willi, Roland, CH-8413 Neftenbach (CH)
(74) Vertreter: Heubeck, Bernhard

(56) Entgegenhaltungen:
- EP-A- 0 021 421
- WO-A-86/03117
- DE-A- 3 314 038
- FR-A- 2 417 293

## Beschreibung

Die Erfindung handelt von einem künstlichen Kniegelenk bestehend aus einem konvexen Femurteil mit einer zylindrischen und mit einer bombierten Kondyle sowie aus einem pfannenartigen Tibiateil, der an dem Femurteil eine Abroll- und Gleitbewegung durchführt, wobei die Führungsfläche für die zylindrische Kondyle in einer Ebene senkrecht zu einer Schaftachse des Tibiateils angeordnet ist.

Eine Kniegelenkprothese mit konvexen Femurteil und mit konkaven Führungsflächen auf dem Tibiateil ist in US-PS 4,568,348 gezeigt. Diese Anordnung hat den Nachteil, dass die Führung des Tibiateils auf eine zur Beugeachse senkrechten Ebene am Femurteil beschränkt ist, indem seitliche Führungsschultern und konkave Führungsflächen am Tibiateil an seitlichen Führungsschultern und konvexen Führungsflächen des Femurteils anliegen.

Die Offenlegungsschrift DE-A-33 14 038 offenbart eine Kniegelenkprothese deren mediale Femurkondyle Kugelform aufweist, während die laterale Kondyle Kreiszylinderform hat. Dadurch, dass die mediale Gegenfläche als eine sphärische Pfanne und die laterale Gegenfläche als eine Ebene quer zur Tibiaachse ausgeführt ist, kann der Tibiateil bei jeder Beugung um die Polachse der sphärischen Pfanne gedreht werden. Ein Nachteil dieser Anordnung besteht darin, dass im gestreckten Zustand, in welchem grosse Gewichtskräfte auftreten, auch grosse Verdrehmomente um die Tibiaachse auftreten, welche abgesehen von der Reibung ausschliesslich vom Bandapparat aufgenommen werden müssen. Ein weiterer Nachteil besteht darin, dass bei allen Beugestellungen unabhängig von der Spannung in den einzelnen Bändern eine Verdrehung um die relativ weit gegen medial liegende Polachse der sphärischen Pfanne vorgenommen werden muss.

Hier schafft die Erfindung Abhilfe. Aufgabe der Erfindung ist es, der anatomischen Wirkungsweise eines natürlichen Kniegelenks möglichst nahe zu kommen. Gemäss der Erfindung wird die Aufgabe durch die kennzeichnenden Merkmale des unabhängigen Anspruchs 1 gelöst.

Die Vorteile der Erfindung bestehen darin, dass das hintere Kreuzband wegen seiner anatomischen Wirkungsweise erhalten bleibt und dass wie beim natürlichen Knie eine geringe Verdrehung in der Schaftachse gegen einen stetig wachsenden Widerstand möglich ist. Die abhängigen Unteransprüche 2 bis 5 beziehen sich auf vorteilhafte Weiterbildungen der Erfindung.

Im folgenden wird die Erfindung anhand von einem Ausführungsbeispiel beschrieben. Es zeigen:
- Figur 1: Die frontale Ansicht eines Femurteils und im Abstand dazu einen Vertikalschnitt durch die am Femurteil geführten Führungsflächen des Tibiateils;
- Figur 2: einen Ausschnitt gemäss Figur 1, in dem der Femurteil und der Tibiateil miteinander in Kontakt sind und vertikal geschnitten sind; und
- Figur 3: eine Draufsicht auf die Führungsflächen des Tibiateils; und
- Figur 4: schematisch einen sagittalen Schnitt durch einen Tibiateil entsprechend Figur 3.

Die Erfindung zeigt ein künstliches Kniegelenk, das aus einem konvexen Femurteil 1 und aus einem pfannenartigen Tibiateil 2 besteht. Der Tibiateil 2 führt am Femurteil 1, welcher mit zunehmender Beugung kleinere Krümmungsradien aufweist, eine Abroll- und Gleitbewegung durch, wobei Femurteil 1 und Tibiateil 2 je eine laterale Führungsfläche F₂, F₂' und je eine mediale Führungsfläche F₁, F₁' aufweisen. Die mediale Führungsfläche F₁' des Tibiateils 2 bildet eine ebene Flachführung, die bei verschiedenen Beugewinkeln unter Linienberührung an zylindrischen Führungsflächen F₁ des Femurteils 1 anliegt, während die laterale Führungsfläche F₂' des Tibiateils bei verschiedenen Beugewinkeln in der frontalen Ebene in lateraler Richtung durch Linienberührung auf Kreissegmenten mit gleichem Krümmungsradius R₃ von der lateralen Kondyle gefangen ist und eine Spur 7 in einer zur medialen Führungsfläche F₁' parallele Ebene bildet. Dabei ist dieser gleiche Krümmungsradius R₃ seinem Betrag nach zwischen dem kleinsten und grössten des in der Spur 7 laufenden Beugeradius Rₙ der lateralen Kondyle.

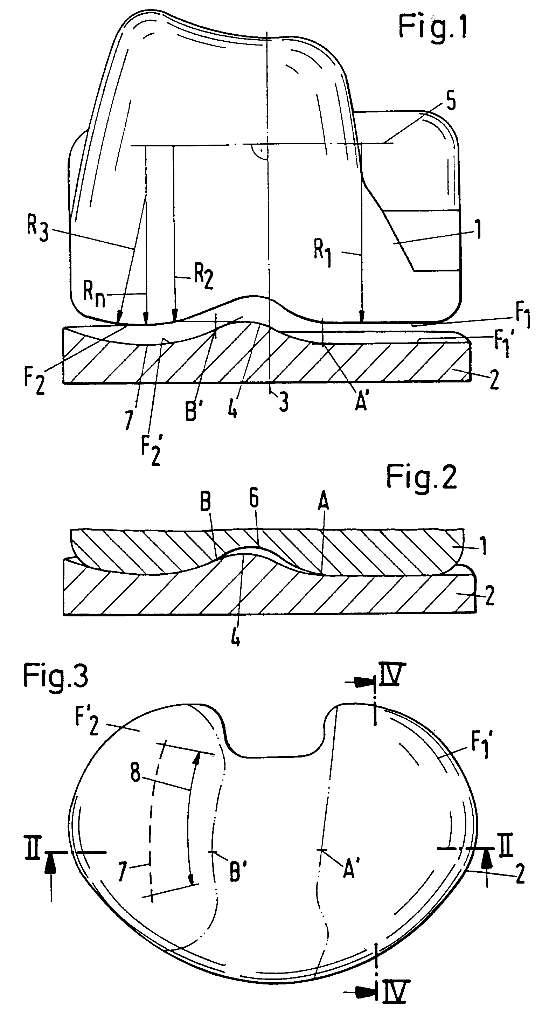

Entsprechend Figur 1 ist die mediale Führungsfläche F₁' des Tibiateils eine ebene Fläche, die senkrecht zur Tibiaachse 3 steht, während die Spur 7 der lateralen Führungsfläche F₂' ebenfalls in einer Ebene senkrecht zur Tibiaachse 3 liegt und in dieser Ebene eine Krümmung um einen Grenzwinkel 8 von 20^{o} gegen lateral aufweist, um eine Innenrotation des Tibiateils 2 um die Tibiaachse 3 zu ermöglichen. Die Erzeugende für die lateralen Führungsflächen ist ein Kreissegment mit Radius R₃, die beim Tibiateil 2 längs der Spur 7 geführt ist und die beim Femurteil 1 für einen bestimmten Beugewinkel jeweils die gleiche Drehachse 5 für ihren Radius Rₙ, welcher der Spur 7 folgt, sowie für ihre allgemeinen Punkte mit Radius R₂ aufweist, wie sie die mediale Führungsfläche F₁ mit Radius R₁ aufweist.

Tibiaseitig wird eine Art Eminentia in einer Uebergangszone 4 zwischen den Grenzpunkten A, A' der medialen Führung und den Grenzpunkten B, B' der lateralen Zone gebildet. Dabei ändert sich die Steigung stetig von medial nach lateral, sodass zwischen Femurteil 1 und Tibiateil 2 ein Zwischenraum 6 entsteht, der die Verdrehung um den Grenzwinkel 8 zulässt.

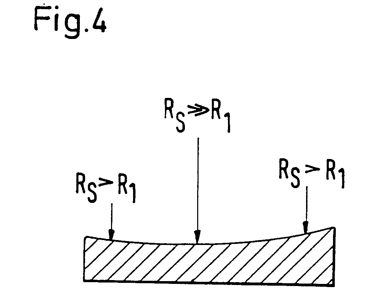

Im sagittalen Schnitt der Figur 4 ist gezeigt, dass die Ebene F₁ und die dazu in einer Parallelebene liegende Spur 7 im sagittalen Randbereich des Tibiateils 2 in einen hochgezogenen Rand übergehen, wobei der Krümmungsradius Rₛ > R₁ resp. Rₛ > Rₙ ist. Dieser Randbereich stellt dann mehr eine Wegbegrenzung für den Femurteil 2 dar. Der mittlere für die Translation vorgesehene Bereich braucht nicht absolut eben zu sein. Er kann eine leichte Krümmung aufweisen, wobei Rₛ >> R₁ resp. Rₛ >> Rₙ ist.

## Patentansprüche

1. Künstliches Kniegelenk bestehend aus einem konvexen Femurteil (1) mit einer zylindrischen und mit einer bombierten Kondyle (F₁, F₂) sowie aus einem pfannenartigen Tibiateil (2), der an dem Femurteil eine Abroll- und Gleitbewegung durchführt, wobei die Führungsfläche für die zylindrische Kondyle (F₁) in einer Ebene (F₁') senkrecht zu einer Schaftachse (3) des Tibiateils (2) angeordnet ist, dadurch gekennzeichnet, dass die Femurkondylen mit zunehmender Beugung kleinere Krümmungsradien (R₁, R₂, Rₙ) aufweisen und dass die zylindrische Kondyle (F₁) und ihre Gegenfläche (F₁') auf der medialen Seite angeordnet sind, während die laterale Kondyle (F₂) und ihre Gegenfläche (F₂') als gemeinsame Erzeugende ein Kreissegment mit Radius (R₃) aufweisen, wobei eine Spur (7) der lateralen Führungsfläche (F₂') in einer Ebene senkrecht zur Schaftachse (3) liegt und eine Krümmung innerhalb von einem Grenzwinkel (8) um die Schaftachse (3) aufweist, um eine geringe Verdrehung zu ermöglichen.

2. Künstliches Kniegelenk nach Anspruch 1, dadurch gekennzeichnet, dass der Radius (R₃) der Erzeugenden in seinem Betrag zwischen dem kleinsten und dem grössten der in der sagittalen Ebene vorkommenden Krümmungsradien (Rₙ) der Kondylenfläche (F₂) liegt, wobei ein Krümmungsradius (Rₙ) ein Radius ist, der jeweils in der Spur (7) läuft.

3. Künstliches Kniegelenk nach Anspruch 1 oder 2, dadurch gekennzeichnet, dass in transversaler Richtung, eine Ubergangszone (4) zwischen lateraler und medialer Führungsfläche (F₂', F₁') des Tibiateils (2) besteht, in der die Steigung vom Wert der lateralen Führungsfläche eines Grenzpunktes (B') eine Art Eminentia bildend stetig in den Wert der medialen Führungsfläche eines Grenzpunktes (A') übergeht.

4. Künstliches Kniegelenk nach Anspruch 3, dadurch gekennzeichnet, dass der Femurteil (1) in unverdrehter Lage von der Uebergangszone (4) des Tibiateils (2) soweit entfernt ist, dass er eine Verdrehung des Tibiateils (2) in der Tibiaachse (3) bis zu einem Grenzwinkel (8) zulässt.

5. Künstliches Kniegelenk nach Anspruch 4, dadurch gekennzeichnet, dass die Verdrehung des Tibiateils (2) in der Tibiaachse (3) bis zu einem Grenzwinkel (8) von 20^{o} erreichbar ist.

6. Künstliches Kniegelenk nach einem der Ansprüche 1 bis 5, dadurch gekennzeichnet, dass die Ebene (F₁') und die dazu in einer Parallelebene liegende Spur (7) in der sagittalen Ebene eine leichte Krümmung mit einem endlichen Krümmungsradius (Rₛ) aufweisen, der ein Vielfaches der Krümmungsradien (R₁, Rₙ) der Führungsflächen (F₁, F₂) des Femurteils (1) beträgt.

7. Künstliches Kniegelenk nach Anspruch 6, dadurch gekennzeichnet, dass die Krümmungsradien (Rₛ) in der sagittalen Ebene sich zum Rand des Tibiateils (2) hin verringern, aber immer noch grösser als der Radius (R₁) resp. (Rₙ) sind.

## Claims

1. An artificial knee joint consisting of a convex femoral part (1) having a cylindrical and having a dished condyle (F₁, F₂) and also of a socket-type tibial part (2), which at the femoral part performs a rolling and sliding movement, with the guide surface for the cylindrical condyle (F₁) being disposed in a plane (F₁') perpendicularly to a shaft axis (3) of the tibial part (2),
**characterised in that** the femur condyles have smaller radii of curvature (R₁, R₂, Rₙ) as flexion increases, **and in that** the cylindrical condyle (F₁) and its counter-face (F₁') are disposed on the medial side, while the lateral condyle (F₂) and its counter-face (F₂') have a circular segment with radius (R₃) as the common generatrix, with a track (7) of the lateral guide face (F₂') lying in a plane perpendicularly to the shaft axis (3) and having a curvature inside a critical angle (8) around the shaft axis (3) in order to enable a slight torsion.

2. An artificial knee joint according to Claim 1,
**characterised in that** the value of the radius (R₃) of the generatrix lies between the smallest and largest of the radii of curvature (Rₙ) of the condyle surface (F₂) present in the sagittal plane, with a radius of curvature (Rₙ) being a radius which extends in each case in the track (7).

3. An artificial knee joint according to Claim 1 or 2,
**characterised in that** in the transversal direction there is a transitional zone (4) between the lateral and medial guide surfaces (F₂', F₁') of the tibial part (2), in which the gradient from the value of the lateral guide surface of a limit point (B') forming a type of eminentia continually changes into the value of the medial guide surface of a limit point (A').

4. An artificial knee joint according to Claim 3,
**characterised in that** in a non-twisted position the femoral part (1) is at such a distance from the transitional zone (4) of the tibial part (2) that it permits a torsion of the tibial part (2) in the tibia axis (3) up to a critical angle (8).

5. An artificial knee joint according to Claim 4,
**characterised in that** the torsion of the tibial part (2) in the tibial axis (3) can be achieved right up to a critical angle (8) of 20°.

6. An artificial knee joint according to one of Claims 1 to 5,
**characterised in that** the plane (F₁') and the track (7) in the sagittal plane which lies in a parallel plane thereto have a slight curvature with a final radius of curvature (Rₛ) which is a multiple of the radii of curvature (R₁, Rₙ) of the guide surfaces (F₁, F₂) of the femoral part (1).

7. An artificial knee joint according to Claim 6,
**characterised in that** the radii of curvature (Rₛ) in the sagittal plane decrease towards the edge of the tibial part (2), but even so are larger than the radius (R₁) or (Rₙ) respectively.

## Revendications

1. Articulation de genou artificielle, constituée d'une partie fémur (1) convexe, ayant un condyle cylindrique et un condyle bombé (F₁, F₂), ainsi que d'une partie tibia (2) en forme de cuvette, qui effectue sur la partie fémur un mouvement de déroulement et de glissement, la surface de guidage destinée au condyle cylindrique (F₁) dans un plan (F₁') étant disposée perpendiculairement à un axe de tige (3) de la partie tibia (2), caractérisée en ce que les condyles de fémur présentent des rayons de courbure (R₁, R₂, Rₙ) allant en diminuant lorsque la flexion augmente, et en ce que le condyle cylindrique (F₁) et sa surface conjuguée (F₁') sont disposés sur la face médiale, tandis que le condyle latéral (F₂) et sa face conjuguée (F₂') présentent, à titre de génératrice commune, un segment de cercle d'un rayon (R₃), une piste (7) de la surface de guidage latérale (F₂') étant située dans un plan perpendiculaire par rapport à l'axe de tige (3) et présente une courbure, dans les limites d'un angle de limitation (8), autour de l'axe de tige (3), afin de permettre une légère rotation.

2. Articulation de genou artificielle selon la revendication 1, caractérisée en ce que le rayon (R₃) de la génératrice a une valeur située entre les valeurs minimale et maximale des rayons de courbure (Rₙ) se présentant dans le plan sagittal pour la surface de condyle (F₂), le rayon de courbure (Rₙ) étant le rayon se présentant spécifiquement dans la piste (7).

3. Articulation de genou artificielle selon la revendication 1 ou 2, caractérisée en ce que, dans la direction transversale, est créée, entre les faces de guidage latérale et médiale (F₂', F₁') de la partie tibia (2), une zone de transition (4) dans laquelle la pente passe de la valeur de la surface de guidage latérale d'un point limite (B'), en constituant un genre d'éminence, continûment, à la valeur de la face de guidage médiale d'un point limite (A').

4. Articulation de genou artificielle selon la revendication 3, caractérisée en ce que la partie fémur (1), dans la position non tournée, est espacée de la zone de transition (4) de la partie tibia (2) d'une distance faisant qu'une rotation de la partie tibia (2) dans l'axe de tibia (3) est possible, jusqu'à l'atteinte de l'angle de limitation (8).

5. Articulation de genou artificielle selon la revendication 4, caractérisée en ce que la rotation de la partie tibia (2) dans l'axe de tibia (3) peut être atteinte jusqu'à un angle limite (8) de 20°.

6. Articulation de genou artificielle selon l'une des revendications 1 à 5, caractérisée en ce que le plan (F₁') et la piste (7) située dans un plan parallèle à celui-ci présentent dans le plan sagittal une légère courbure ayant un rayon de courbure final (Rₛ) qui est un multiple des rayons de courbure (R₁, Rₙ) des surfaces de guidage (F₁, F₂) de la partie fémur (1).

7. Articulation de genou artificielle selon la revendication 6, caractérisée en ce que les rayons de courbure (Rₛ) vont en diminuant, dans le plan sagittal, jusqu'au bord de la partie tibia (2), mais sont encore toujours supérieurs aux rayons de courbure (R₁), respectivement (Rₙ).
